# EUROPEAN PATENT APPLICATION

(11) **EP 2 609 906 A1**
(43) Date of publication of application: **03.07.2013**
(21) Application number: 11196099.3
(22) Date of filing: 29.12.2011
(51) Int. Cl.: A61K 8/60, A61Q 5/10

(54) **Hair dyeing composition**

(71) Applicant: KPSS-Kao Professional Salon Services GmbH, 64297 Darmstadt (DE)
(72) Inventor: Molenda, Michael, 60318 Frankfurt (DE); Lipinski, Normen, 60325 Frankfurt (DE)
(74) Representative: Grit, Mustafa

(57) **Abstract**

Present invention relates to a hair dyeing composition comprising an anionic sugar surfactant which delivers long lasting colouration to human hair. The object of the present invention is an aqueous hair dyeing composition comprising one or more oxidative dyes and at least one anionic sugar surfactant. Suitable and particularly preferred anionic sugar surfactant is lauryl glucose carboxylate and its alkali metal salts such as sodium and potassium.

## Description

Present invention relates to a hair dyeing composition comprising an anionic sugar surfactant which delivers long lasting colouration to human hair.

Lifetime of a hair colour has always been one of the important point in developing hair colour products and/or products to be used on artificially coloured hair. Especially the lifetime of colour achieved with a composition comprising hair direct dyes is always considered to be short. It may be that the problem is not as aggravated as in the case of direct dyes but there are similar problems with oxidative dyes as well. There have been many attempts to extend the lifetime of colour, but without a satisfactory result up until now.

The aim of the inventors of the present invention is to provide hair dyeing compositions comprising oxidative dyes which deliver hair color with considerable longer life time compared to the ones currently available.

The inventors of the present invention have surprisingly found out that a hair dyeing composition comprising one or more oxidative dyes and an anionic sugar surfactant has considerably longer lifetime than a composition based on hair direct dyes but not comprising anionic sugar surfactant.

Accordingly the first object of the present invention is an aqueous hair dyeing composition comprising one or more oxidative dyes and at least one anionic sugar surfactant.

The term sugar surfactant it is meant that the surfactant is derived from a monosaccharide such as a hexose for examples glucose, galactose and mannose.

Further object of the present invention is the use of an aqueous composition comprising one or more oxidative dyes and at least one anionic sugar surfactant for colouring hair.

Still further object of the present invention is a process for colouring hair wherein an aqueous composition comprising one or more oxidative dyes and at least one anionic sugar surfactant is mixed with a composition comprising at least one oxidizing agent and applied onto hair and rinsed off from hair after processing 1 to 45 min.

Further object of the present invention is a kit for hair comprising one or more compositions wherein one of the compositions is an aqueous composition comprising one or more oxidative dyes and at least one anionic sugar surfactant and the other composition comprising at least one oxidizing agent preferably hydrogen peroxide.

Compositions of the present invention comprise one or more oxidative dyes. The term oxidative dye refers primarily oxidative dye precursors but when an oxidative dye precursor is present does not exclude coupling substances either. Suitable oxidative dyestuffs precursors are tetraaminopyrimidines, in particular 2,4,5,6-tetraaminopyrimidine and the lower alkyl derivatives thereof; suitable triaminohydroxypyrimidines are, for example 4-hydroxy-2,5,6-triaminopyrimidine, 2-hydroxy-4,5,6-triaminopyrimidine and 5-hydroxy-2,4,6-triaminopyrimidine; suitable mono- and diamino dihydroxypyrimidines are, for example, 2,6-dihydroxy-4,5-diaminopyrimidine, 2,4-diamino-6-hydroxy-pyrimidine or 4,6-dihydroxy-2,5-diaminopyrimidine or the water-soluble salts thereof, aminophenol derivatives such as 4-aminophenol, 4-amino-3-methylphenol, 2-chloro-4-aminophenol, 2,6-dichloro-4-aminophenol, 2,4-diamino-phenol, 2,6-dibromo-4-aminophenol and/or 2-aminophenol and water-soluble salts thereof, furthermore, phenylenedimanine derivatives such as 2,5-diamino-toluene, 2-n-propyl or 2-ethyl-p-phenylene-diamine, 2,6-dimethyl-p-phenylenediamine, 2-(2,5-diaminophenyl) ethanol, 1-amino -4-bis-(2'-hydroxy-ethyl)aminobenzene, 2-(2-hydroxyethyl amino)-5-aminotoluene, 4,4'-diaminodiphenylamine, 4-aminodiphenylamine, 2-amino-5-N,N-diethyl aminotoluene, 4-amino-N-ethyl-N-isopropyl aniline, 2-chloro-p-phenylenediamine, 1-β-hydroxyethyl-2,5-diamino-4-chlorobenzene, 1-β-hydroxyethyl-2,5-diamino-4-methyl benzene, 2-methoxy-p-phenylenediamine, N,N-diethyl-p-phenylenediamine, 1-amino-4-β-methoxyethyl aminobenzene, 1-dimethyl-amino-4-aminobenzene, 1-hydroxy-2,5-diamino-4-methyl benzene, 1-hydroxymethyl-2,5-diaminobenzene, 1,3-dimethyl-2,5-diaminobenzene, 1,4-diamino isopropyl benzene and/or 1-amino-4-β-hydroxypropyl aminobenzene or the water-soluble salts thereof, pyrazole derivatives such as 1-hydroxyethyl-4,5-diaminopyrazole, 3,4-diamino-5-hydroxypyrazole, 3,5-diaminopyrazole, 3,5-diamino pyrazol-1-carboxamide, 3-amino-5-hydroxypyrazole, 1-phenyl-2-methylpyrazole, 1-phenyl-3-methylpyrazole-5-one, 3,5-dimethylpyrazole, 3,5-dimethylpyrazole-1-methanol, 1-methyl-4,5-diaminopyrazole, 1-methylethyl-4,5-diaminopyrazole, 1-phenylmethyl-4,5-diaminopyrazole, 1-methyl-4,5-diaminopyrazole, 1-(4-methylphenyl)methyl-4,5-diaminopyrazole, 1-methyl-3-phenyl-4,5-diaminopyrazole and the water-soluble salts. The use of the above mentioned oxidative dye precursors as mixture is also customary in hair coloring area.

The composition according to the invention comprises additionally at least one coupling substance. Suitable ones are resorcinol, 2-methyl resorcinol, 4-chlororesorcinol, 2-amino-4-chlorophenol, 5-amino-4-methoxy-2-methylphenol, 3-amino-phenol, 1-methyl-2-hydroxy-4-aminobenzene, 3-N,N-dimethyl aminophenol, 2.6-dihydroxy-3.5-dimethoxypyridine, 5-amino-3-methylphenol, 6-amino-3-methylphenol, 3-amino-2-methylamino-6-methoxypyridine, 2-amino-3-hydroxy-pyridine, 2-dimethyl-amino-5-aminopyridine, 2,6-diaminopyridine, 1,3-diamino- benzene, 1-amino-3-(2'-hy-droxyethylamino)benzene, 1-amino-3-[bis(2'-hydroxy-ethyl) amino]benzene, α-naphthol, 4,6-dichlororesorcinol, 1,3-diamino-toluene, 1-hydroxy naphthalene, 4-hydroxy-1,2-methylenedioxy benzene, 1,5-dihydroxy naphthalene, 1,6-dihydroxy naphthalene, 1,7-dihydroxy naphthalene, 2,7-dihydroxy naphthalene, 1-hydroxy-2-methyl naphthalene, 4-hydroxy-1.2-methyldioxy benzene, 2,4-diamino-3-chlorophenol, 5-amino-2-methoxyphenol and/or 1-methoxy-2-amino-4-(2'-hydroxyethyl amino)benzene or the water-soluble salts thereof. However, this shall not exclude the addition of further developing and coupling substances. In the preferred embodiment of the present invention composition comprise additionally at least one coupling agent.

The weight proportion of the named developing substances to the coupling substances ranges between about 1 : 8 to 8 : 1, preferably about 1 : 5 to 5 : 1, in particular 1 : 2 to 2 : 1. In the hair dyeing compositions according to the invention, the coupling substance(s) as reaction partners of the developing substance(s) are present in approximately the same molecular proportions as the developing substances. Total concentration of one or more oxidative dyestuff is in the range of 0.01 % to 10.0 %, preferably 0.05 % to 8 %, in particular 0.1 % to 5 % by weight, calculated to the total composition.

Compositions of the present invention may comprise one or more hair direct dyes. The direct dyes suitable are the ones generally known in the art such as anionic, cationic and nonionic ones. Plant dyes are also suitable for the compositions of the present invention.

Suitable anionic direct dyes in aqueous composition are Acid Black 1, Acid Blue 1, Acid Blue 3, Food Blue 5, Acid Blue 7, Acid Blue 9, Acid Blue 74, Acid Orange 3, Acid Orange 6, Acid Orange 7, Acid Orange 10, Acid Red 1, Acid Red 14, Acid Red 18, Acid Red 27, Acid Red 50, Acid Red 52, Acid Red 73, Acid Red 87, Acid Red 88, Acid Red 92, Acid Red 155, Acid Red 180, Acid Violet 9, Acid Violet 43, Acid Violet 49, Acid Yellow 1, Acid Yellow 23, Acid Yellow 3, Food Yellow No. 8, D&C Brown No. 1, D&C Green No. 5, D&C Green No. 8, D&C Orange No. 4, D&C Orange No. 10, D&C Orange No. 11, D&C Red No. 21, D&C Red No. 27, D&C Red No. 33, D&C Violet 2, D&C Yellow No. 7, D&C Yellow No. 8, D&C Yellow No. 10, FD&C Red 2, FD&C Red 40, FD&C Red No. 4, FD&C Yellow No. 6, FD&C Blue 1, Food Black 1, Food Black 2, Disperse Black 9 and Disperse Violet 1 and their alkali metal salts such as sodium, potassium. Among those, the most preferred anionic dyestuffs are Acid Red 52, DC Violet 2, DC Red 33, DC Orange 4, DC Red 27 and DC Yellow 10.

Suitable cationic dyes in aqueous composition are in principal those available on the market for cosmetic hair colouring applications. For this purpose, special reference is made to the PCT application WO 95/15144 of Ciba-Geigy AG. Some examples to those are Basic Blue 6, Basic Blue 7, Basic Blue 9, Basic Blue 26, Basic Blue 41, Basic Blue 99, Basic Brown 4, Basic Brown 16, Basic Brown 17, Natural Brown 7, Basic Green 1, Basic Red 2, Basic Red 12 Basic Red 22, Basic Red 76, Basic Violet 1, Basic Violet 2, Basic Violet 3, Basic Violet 10, Basic Violet 14, Basic Yellow 57, Basic red 51, Basic Yellow 87 and Basic Orange 31. The most preferred ones are Basic red 51, Basic Yellow 87 and Basic Orange 31 sold by CIBA.

Additionally, the aqueous compositions of the present invention comprise neutral dyes (HC dyes), so called nitro dyes. Some examples to those are: HC Blue No.2, HC Blue No.4, HC Blue No.5, HC Blue No.6, HC Blue No.7, HC Blue No.8, HC Blue No.9, HC Blue No.10, HC Blue No.11, HC Blue No.12, HC Blue No.13, HC Brown No.1, HC Brown No.2, HC Green No.1, HC Orange No.1, HC Orange No.2, HC Orange No.3, HC Orange No.5, HC Red BN, HC Red No.1, HC Red No.3, HC Red No.7, HC Red No.8, HC Red No.9, HC Red No.10, HC Red No.11, HC Red No.13, HC Red No.54, HC Red No.14, HC Violet BS, HC Violet No.1, HC Violet No.2, HC Yellow No.2, HC Yellow No.4, HC Yellow No.5, HC Yellow No.6, HC Yellow No.7, HC Yellow No.8, HC Yellow No.9, HC Yellow No.10, HC Yellow No.11, HC Yellow No.12, HC Yellow No.13, HC Yellow No.14, HC Yellow No.15, 2- Amino-6-chloro-4-nitrophenol, picramic acid, 1,2-Diamino-4-nitrobenzol, 1,4-Diamino-2-nitrobenzol, 3-Nitro-4-aminophenol, 1-Hydroxy-2-amino-3-nitrobenzol and 2-hydroxyethylpicramic acid.

Plant dyestuffs can also be used alone or in combination with synthetic direct-acting dyestuffs, for example henna (red or black), alkanna root, laccaic acid, indigo, logwood powder, madder root and rhubarb powder, etc.

Further suitable direct dyes which are anionic under alkaline conditions are according to the following structures:

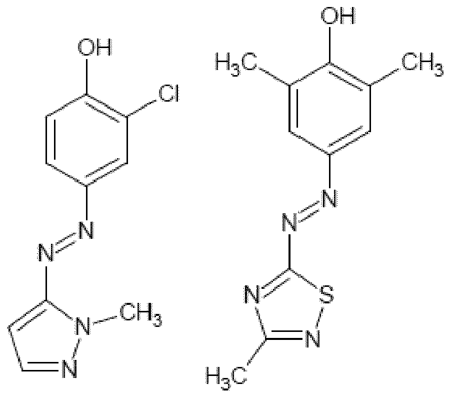

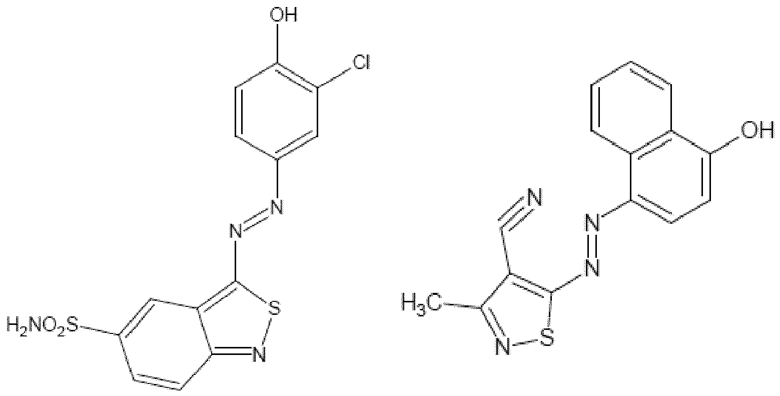

From the above disclosed direct dyes the preferred are cationic and nitro dyes.

According to the invention, the composition comprises one or more direct dyes at a concentration of 0.01 to 10%, preferably 0.05 to 7.5% and more preferably 0.2 to 5% by weight calculated to the total composition. The composition can also comprise mixture of several direct dyes i.e. an anionic, a cationic and/or a nonionic ones. In such a case the dyes may be mixed at any ratio with each other.

Compositions of the present invention comprise at least one at least one anionic sugar surfactant. Suitable and particularly preferred anionic sugar surfactant is lauryl glucose carboxylate and its alkali metal salts such as sodium and potassium. Lauryl glucose carboxylate is carboxymethyl ether of lauryl glucoside. Sodium lauryl glucose carboxylate is available under the trade name Plantapon from Cognis.

Compositions comprises at least one sugar surfactant, in particular lauryl glucose carboxylate and its alkali metal salt at a concentration in the range of 0.1 to 25%, preferably 0.2 to 20 and more preferably 0.5 to 15 and most preferably 1 to 10% by weight calculated to the total composition.

Compositions of the present invention have a pH in the range of 5 to 12 and preferably 6 to 11, more preferably 6.8 to 10.5 and in particular 8 to 10.

Compositions preferably comprise at least one alkalizing agent. Suitable alkalizing agents are ammonia or ammonium hydroxide and a compound according to the general formula

R₁R₂R₃N

wherein R₁, R₂ and R₃ are same or different H, C1 - C6 alkyl, C1 - C6 monohydroxyalkyl or C2 - C6 polyhydroxyalkyl with the condition that at least one of R₁, R₂ and R₃ is a mono or polyhydroxyalkyl. Preferably R₁, R₂ and R₃ are same or different H, C1 - C4 alkyl, C1 - C4 monohydroxyalkyl or C2 - C4 polyhydroxyalkyl with the condition that at least one of R₁, R₂ and R₃ is a mono or polyhydroxyalkyl.

Suitable alkanolamines according to the general formula of above are monoethanolamine, diethanolamine, triethanolamine, monoethanol methylamine, monoethanoldimethylamine, di-ethanolmethylamine, monoethanolethylamine, monoethanoldiethylamine, diethanolethylamine, monoethanolpropylamine, monoethanoldipropylamine, diethanolpropylamine, monoethanolbutylamine and diethanolbutylamine.

Preferred are monoethanolamine, diethanolamine and triethanolamine. The most preferred is monoethanolamine. Ammonia is also preferred as an alkalizing agent.

The concentration of at least one alkalizing agent varies between 1 and 35%, preferably 1 and 30, more preferably 2.5 and 25 and most preferably 2.5 to 20% by weight calculated to the total composition.

Composition of the present invention preferably comprises at least one oxidizing agent. The oxidizing agents suitable are hydrogen peroxide, urea peroxide, melamin peroxide or perborate salts. The preferred oxidizing agent is hydrogen peroxide, at a concentration in a range of 2 to 12 % by weight calculated to the total composition.

Compositions of the present invention may be in the form of emulsion, solution, dispersion and/or gel. Emulsion is the preferred form.

In the case the composition is in the form of an emulsion, it comprises as an emulsion base at least one fatty alcohol or mixture of fatty alcohols with the chain length of 14 to 22 C atoms. Examples to suitable fatty alcohols, without limiting the choice, are myristyl alcohol, cetyl alcohol, stearyl alcohol, behenyl alcohol and cetostearyl alcohol. The most preferred is cetostearyl alcohol well known with its trade name Lanette O or as Lanette N in mixture with sodium cetearyl sulfate from Cognis.

The concentration of fatty alcohol(s) is in the range from 0.5 to 20%, preferably 1 to 15% by weight, calculated to total composition prior to mixing with oxidizing and bleaching and/or highlighting composition.

Compositions according to present invention may comprise surfactants selected from anionic, nonionic, amphoteric and cationic surfactants as emulsifier or solubilizer. Cationic surfactants are as well used as hair conditioners in the compositions.

Anionic surfactants suitable within the scope of the invention are in principal known from the cleansing compositions

These are anionic surfactants of the sulfate, sulfonate, carboxylate and alkyl phosphate type, for example, the known C₁₀-C₁₈-alkyl sulfates, and in particular the respective ether sulfates, for example, C₁₂-C₁₄-alkyl ether sulfate, lauryl ether sulfate, especially with 1 to 4 ethylene oxide groups in the molecule, monoglyceride (ether) sulfates, fatty acid amide sulfates obtained by ethoxylation and subsequent sulfatation of fatty acid alkanolamides, and the alkali salts thereof, as well as the salts of long-chain mono- and dialkyl phosphates.

Additional anionic surfactants useful within the scope of the invention are α-olefin sulfonates or the salts thereof, and in particular alkali salts of sulfosuccinic acid semiesters, for example, the disodium salt of monooctyl sulfosuccinate and alkali salts of long-chain monoalkyl ethoxysulfosuccinates.

Suitable surfactants of the carboxylate type are alkyl polyether carboxylic acids and the salts thereof as well as alkyl amido polyether carboxylic acids and salts thereof. Such products have been known for some time and are on the market, for example, under the trade name "AKYPO^{®}" and "AKYPO-SOFT^{®}".

Also useful are C₈-C₂₀-acyl isethionates, alone or in admixture with other anionic surfactants, as well as sulfofatty acids and the esters thereof.

Further suitable anionic surfactants are also C₈-C₂₂-acyl aminocarboxylic acids or the water-soluble salts thereof. Especially preferred is N-lauroyl glutamate, in particular as sodium salt, as well as, for example, N-lauroyl sarcosinate, N-C₁₂-C₁₈-acyl asparaginic acid, N-myristoyl sarcosinate, N-oleoyl sarcosinate, N-lauroyl methylalanine, N-lauroyl lysine and N-lauroyl aminopropyl glycine, preferably in form of the water-soluble alkali or ammonium, in particular the sodium salts thereof, preferably in admixture with the above-named anionic surfactants.

It is also possible to use mixtures of several anionic surfactants.

An overview of the anionic surfactants suitable for the present invention can furthermore be found in the monography of K. Schrader, "Grundlagen und Rezepturen der Kosmetika", 2nd Ed.(1989, Hüthig Buchverlag), pp. 595-600 and pp. 683 to 691.

Further surfactants in the compositions according to the invention are nonionic surfactants alone or in admixture with anionic surfactants. These are described as well in Schrader, I.c., on pages 600-601 and pp. 694-695. Especially suited nonionic surfactants are, for example, long-chain fatty acid mono- and dialkanolamides, such as coco fatty acid mono- or diethanolamide and myristic fatty acid mono or diethanolamide, stearic acid mono or diethanolamide. Further nonionic surfactants suited are alkyl polyglucosides with an alkyl group of 8 to 18 carbon atoms, and with 1 to 5 glucoside units. Further additionally useful nonionic surfactants are, for example, the various sorbitan esters, such as polyethylene glycol sorbitan stearic acid ester, fatty acid polyglycol esters or polycondensates of ethyleneoxide and propyleneoxide, as they are on the market, for example, under the trade name "Pluronics^{R}", as well as fatty alcohol ethoxylates. Further nonionic surfactants preferred in the dyeing compositions according to invention are C₁₀-C₂₂-fatty alcohol ethoxylates. Especially suited are C₁₀-C₂₂-fatty alcohol ethers, the alkyl polyglycol ethers known by the generic terms "Laureth", "Myristeth", "Oleth", "Ceteth", "Deceth", "Steareth" and "Ceteareth" according to the CTFA nomenclature, including addition of the number of ethylene oxide molecules, e.g., "Laureth-16": The average degree of ethoxylation thereby ranges between about 2.5 and about 25, preferably about 10 and about 20.

As further surfactant component, the compositions according to the invention can also contain amphoteric or zwitterionic surfactants. Useful as such are in particular the various known betaines such as alkyl betaines, fatty acid amidoalkyl betaines and sulfobetaines, for example, lauryl hydroxysulfobetaine; long-chain alkyl amino acids, such as cocoaminoacetate, cocoaminopropionate and sodium cocoamphopropionate and -acetate have also proven suitable.

Composition can contain cationic surfactants as emulsifier, solubilizer and/or conditioning ingredients according to the formula 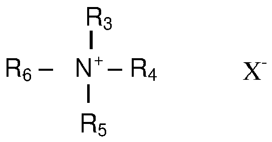 where R₃ is a saturated or unsaturated, branched or linear alkyl chain with 8-22 C atoms or

R₇ CO NH (CH₂)ₙ

where R₇ is saturated or unsaturated, branched or linear alkyl chain with 7-21 C atoms and n has typical value of 1 - 4 or

R₈ CO O (CH₂)ₙ

where R₈ is saturated or unsaturated, branched or linear alkyl chain with 7-21 C atoms and n has typical value of 1 - 4, and
R₄ is H or unsaturated or saturated, branched or linear alkyl chain with 1 - 22 C atoms or

R₇ CO NH (CH₂)ₙ

or

R₈ CO O (CH₂)ₙ

where R₇, R₈ and n are same as above.

R₅ and R₆ are H or lower alkyl chain with 1 to 4 Carbon atoms, and X is typically chloride, bromide, methosulfate.

Typical examples of those ingredients are cetyl trimethly ammonium chloride, stear trimonium chloride, dipalmitoyl dimonium chloride, distearyl dimethyl ammonium chloride, stearamidopropyl trimonuim chloride, dioleoylethyl dimethyl ammonium methosulfate, dioleoylethyl hydroxyethylmonium methosulfate.

Concentration of one or more surfactants other than the sugar surfactant in dyeing composition is in the range of 0.1 to 20%, preferably 0.2 to 15% and most preferably 0.2 - 10% by weight, calculated to the total composition.

Further, compositions may comprise polymers selected from the group consisting of cellulose polymer compounds, alginate, polysaccarides and acrylic acid polymers, preferably methyl cellulose compounds, ethyl cellulose compounds, hydroxyethylcellulose compounds, methylhydroxyethylcellulose compounds, methylhydroxypropylcellulose compounds, carboxymethyl cellulose compounds, alginic acids, sodium alginates, ammonium alginates, calcium alginates, gum arabic, guar gum or xanthan gum, or acrylic acid polymers with molecular weights from about 1,250,000 to 4,000,000, alone or in combination with each other. The polymers are used in a total concentration of 0.1 to 15 %, preferably from 0.2 to 10 %, and more preferably in an amount of from 0.5 to 7.5% by weight, calculated to total composition.

Composition can also comprise cationic polymers as conditioning and/or thickening agents. Those are cationic cellulose type polymers know as Polymer JR type from Amerchol such as Polyquaternium 10 or cationic guar gum known with trade name Jaguar from Rhône-Poulenc and chemically for example Guar hydroxypropyl trimonium chloride. Furthermore, chitosan and chitin can also be included in the compositions as cationic natural polymers.

Furthermore, it has been found suitable those cationic polymers known with their CTFA category name Polyquaternium. Typical examples of those Polyquaternium 4, Polyquaternium 6, Polyquaternium 7, Polyquaternium 10, Polyquaternium 11, Polyquaternium 16, Polyquaternium 22, Polyquaternium 24, Polyquaternium 28, Polyquaternium 30, Polyquaternium 37, Polyquaternium 36, Polyquaternium 46, Polyquaternium 67, and Polyquaternium 72.

As well those polymers known with their CTFA category name Quaternium are suitable. Those are for example Quaternium-8, Quaternium-14, Quaternium-15, Quaternium-18, Quaternium-22, Quaternium-24, Quaternium-26, Quaternium-27, Quaternium-30, Quaternium-33, Quaternium-53, Quaternium-60, Quaternium-61, Quaternium-72, Quaternium-78, Quaternium-80, Quaternium-81, Quaternium-82, Quaternium-83 and Quaternium-84.

Compositions of the present invention can comprise an organopolysiloxane polymers wherein at least one silicium atom is linked to an alkylene group having a hetero-atom, in particular a nitrogen atom, with a poly-(N-acyl alkyleneimine) units of the formula 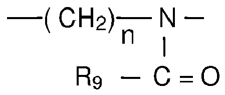 wherein n is a number from 1 to 5 and R₉ is hydrogen, a C₁-C₁₂-alkyl or cycloalkyl, aralkyl or aryl group. Preferred organopolysiloxane polymers are those of the type disclosed in EP-A 640 643, in particular optionally quaternized aminoalkyl, in particular aminopropyl dimethyl polysiloxane/polyethyl oxazoline copolymers of the formula 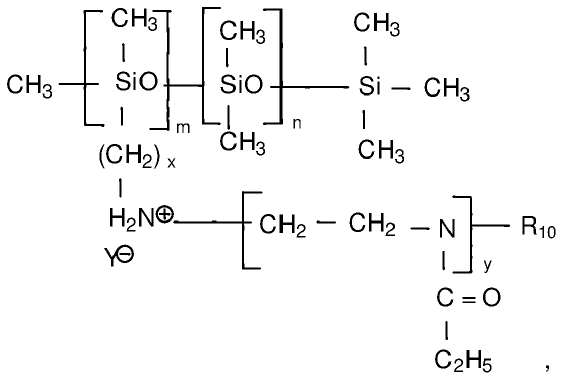 wherein m and n each are numbers from 20 to 10,000, in particular 50 to 7,000, especially 100 to 5,000, x is a number between 1 and 5, preferably 3, and y is a number from 5 to 30, R₁₀ is a C₁-C₁₂-alkyl or aryl group, in particular a methyl, ethyl or benzyl group, and Y⁻ is an anion.

Especially suited are the organopolysiloxanes disclosed under the terms A-1, A-2 and A-3 on pages 12 to 13 of EP-A 640 643. The proportion of graft copolymers in the hair colouring compositions according to the invention ranges from 0.05% to 5%, preferably 0.1 % to 2.5%, in particular 0.5% to 1.5% by weight, calculated to the total composition.

Typical concentration range for any of the cationic polymers as conditioners mentioned above can be 0.1 - 7.5% by weight, preferably 0.3 - 5% by weight and more preferably 0.5 - 2.5% by weight, calculated to total composition

Composition of the present invention may further comprise lipophilic ingredients such as vegetable oils, for example, marula oil, argan oil, shea butter oil, jojoba oil or any other; petrolatum liquid paraffins, especially paraffinum perliquidum and parafiinum subliquidum; silicone oils; hydropobic fatty acid esters such as octyl palmitate, isocetyl palmitate, isopropyl palmitate and octyl stearate, C₁₀- to C₃₆-fatty acid triglycerides, as well as their mixtures. In the case that the use is wished among those the most preferred ones are silicone oils, jojoba oil, fatty acid esters, paraffin oils, combinations of fatty acid esters and paraffin oils. Fatty acid esters and/or paraffin oils and/or silicone oils are particularly preferred. Concentration of these lipophilic compounds are used in a total amount of about 0.1 to 20 percent by weight, preferably from 1 to 15 percent by weight, and more preferably from 2 to 10 percent by weight, calculated to total composition.

In principal any silicone oil is useful as a lipophilic compound. Preferred are arylated silicones as a lipophilic ingredient at a concentration range of 0.1 to 50%, preferably 0.5 to 40% more preferably 1 to 35% and most preferably 2.5 to 30% by weight calculated to total composition.

Another preferred compound in the composition of present invention especially in bleaching and/or highlighting composition and in dyeing composition is ceramide type of compounds according to general formula 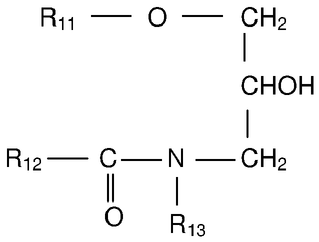 where R₁₁ and R₁₂ are independent from each other alkyl- or. alkenyl group with 10 to 22 carbon atoms, R₁₃ is alkyl or hydroxyl alkyl with 1 to 4 carbon atoms group and n is a number between 1 to 6, preferably 2 or 3. Preferred compound according to the above chemical structure is cetyl-PG-hydroxyethylpalmitamide. Concentration of ceramide type of compounds ranges from 0.01 to 2%, preferably 0.01 to 1% yb weight calculated to total composition before mixing.

The compositions of the present invention can comprise of at least one ubiquinone of the formula (I) 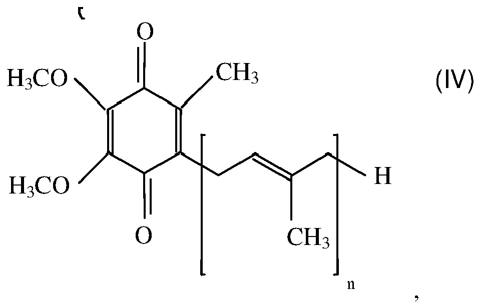 wherein n is a number from 1 to 10. Concentration of ubichinone can vary between 0.001 % and 10 % by weight, calculated to the total composition before mixing.

Compositions may further comprise additional substances found in coloring compositions for hair such as fragrance, humectants, chelants, organic solvents and radical scavangers.

The invention is illustrated with the following examples but not limited to.

### Example 1

| | % by weight |
|---|---|
| p-toluenediamine sulphate | 1.0 |
| 4-Chlorresorcinol | 0.3 |
| Resorcinol | 0.1 |
| m-aminophenol | 0.1 |
| Sodium lauryl glucose carboxylate* | 2.0 (active matter) |
| Lauryl glucoside* | 1.5 (active matter) |
| Ammonia (25%) | 8.0 |
| Water | to 100 |

| | |
|---|---|
| * Plantapon LGC Sorb was used. | |

For comparative purpose, the same composition as above was produced only with lauryl glucoside but not comprising any sodium lauryl glucose carboxylate. The sodium lauryl glucose carboxylate content was replaced with water.

Colouring was carried out applying a composition obtained from mixing the above composition at a weight ratio of 1:1 with a composition comprising hydrogen peroxide at a concentration of 9% by weight and after processing of 30 min at 40°C the tresses were rinsed off with water. It was observed that colour intensity of both tresses were comparable.

The coloured tresses were washed with commercially available shampoo composition under the brand Goldwell and designed for coloured hair for 12 times and that tresses were evaluated by naked eye for their colour intensity. It was observed the tress coloured with inventive composition was clearly darker natural brown coloured than the tress coloured with comparative composition.

Similar results were observed with the following examples

### Example 2

| | % by weight |
|---|---|
| p-toluenediamine sulphate | 0.8 |
| 4-Chlorresorcinol | 0.3 |
| Resorcinol | 0.1 |
| m-aminophenol | 0.1 |
| Basic red 51 | 0.3 |
| Sodium lauryl glucose carboxylate* | 2.0 (active matter) |
| Lauryl glucoside* | 1.5 (active matter) |
| Cetearyl alcohol | 5.0 |
| Ceteareth-20 | 1.0 |
| Monoethanolamine | 7.0 |
| Water | to 100 |

| | |
|---|---|
| * Plantapon LGC Sorb was used. | |

### Example 3

| | % by weight |
|---|---|
| p-toluenediamine sulphate | 0.8 |
| 4-Chlorresorcinol | 0.3 |
| Resorcinol | 0.1 |
| m-aminophenol | 0.1 |
| HC red 3 | 0.3 |
| Sodium lauryl glucose carboxylate* | 1.0 (active matter) |
| Lauryl glucoside* | 0.75 (active matter) |
| Cetearyl alcohol | 4.0 |
| Ceteareth-20 | 1.2 |
| Polyquaternium-10 | 0.5 |
| Ammonia 25% | 8.0 |
| Water | to 100 |

| | |
|---|---|
| * Plantapon LGC Sorb was used. | |

### Example 4

| | % by weight |
|---|---|
| p-toluenediamine sulphate | 0.8 |
| 4-Chlorresorcinol | 0.3 |
| Resorcinol | 0.1 |
| m-aminophenol | 0.1 |
| Acid red 52 | 0.5 |
| Sodium lauryl glucose carboxylate* | 2.0 (active matter) |
| Lauryl glucoside* | 1.5 (active matter) |
| Cetearyl alcohol | 5.0 |
| Ceteareth-20 | 1.0 |
| Polyquaternium-10 | 0.5 |
| Monoethanolamine | 8.0 |
| Water | to 100 |

| | |
|---|---|
| * Plantapon LGC Sorb was used. | |

### Example 5

| | % by weight |
|---|---|
| p-toluenediamine sulphate | 0.3 |
| Resorcinol | 0.1 |
| 4-Hydroxypropylamino-3-nitrophenol | 3.0 |
| 2-Amino-6-chloro-4-nitrophenol | 0.8 |
| HC red 3 | 0.3 |
| Sodium lauryl glucose carboxylate* | 2.0 (active matter) |
| Lauryl glucoside* | 1.5 (active matter) |
| Cetearyl alcohol | 9.0 |
| Ceteareth-20 | 2.0 |
| Cocamide-MEA | 5.6 |
| Sodium cetearyl sulphate | 3.0 |
| Ammonium hydroxide (25%) | 8.0 |
| Water | to 100 |

| | |
|---|---|
| * Plantapon LGC Sorb was used. | |

## Claims

1. An aqueous hair dyeing composition **characterized in that** it comprises one or more oxidative dyes and at least one anionic sugar surfactant.

2. The composition according to claim 1 **characterized in that** at least one anionic sugar surfactant is derived from a monosaccharide such as a hexose, preferably glucose, galactose and mannose, more preferably from glucose.

3. The composition according to claims 1 and 2 **characterized in that** at least one anionic sugar surfactant is lauryl glucose carboxylate and/or its alkali metal salt and preferably it is comprised at a concentration in the range of 0.1 to 25% by weight calculated to total composition.

4. Composition according to any of the preceding claims it comprises at least one oxidative dye precursor and at least one coupling substance.

5. The composition according to any of the preceding claims **characterized in that** it comprises at least one oxidizing agent preferably hydrogen peroxide.

6. The composition according to any of the preceding claims **characterized in that** it comprises at least one alkalizing agent selected from ammonia or ammonium hydroxide and a compound according to the general formula
R₁R₂R₃N
wherein R₁, R₂ and R₃ are same or different H, C1 - C6 alkyl, C1 - C6 monohydroxyalkyl or C2 - C6 polyhydroxyalkyl with the condition that at least one of R₁, R₂ and R₃ is a mono or polyhydroxyalkyl. Preferably R₁, R₂ and R₃ are same or different H, C1 - C4 alkyl, C1 - C4 monohydroxyalkyl or C2 - C4 polyhydroxyalkyl with the condition that at least one of R₁, R₂ and R₃ is a mono or polyhydroxyalkyl.

7. The composition according to any of the preceding claims **characterized in that** it comprises one or more alkalizing agent at a concentration in the range of 1 to 25% by weight calculated to total composition.

8. The composition according to any of the preceding claims **characterized in that** it comprises monoethanolamine as an alkalizing agent.

9. The composition according to any of the preceding claims **characterized in that** it has a pH between 5 and 12.

10. The composition according to any of the preceding claims **characterized in that** it comprises one or more hair direct dyes, preferably selected from anionic, cationic and nonionic nitro dyes.

11. The composition according to any of the preceding claims **characterized in that** it comprise additionally at least one nonionic surfactant preferably alkyl glucoside.

12. The composition according to any of the preceding claims **characterized in that** it is an emulsion and comprises fatty alcohol.

13. The composition according to any of the preceding claims **characterized in that** it comprises at least one hair conditioning agent such as cationic polymer.

14. Process form colouring hair wherein a composition comprising one or more oxidative dyes and at least one anionic sugar surfactant is mixed with a composition comprising at least one oxidizing agent and applied onto hair and rinsed off from hair after processing 1 to 45 min.

15. A kit for hair comprising one or more compositions wherein one of the compositions comprises one or more oxidative dyes and at least one anionic sugar surfactant and another composition comprises at least one oxidizing agent, preferably hydrogen peroxide.
